# EUROPEAN PATENT SPECIFICATION

(11) **EP 1 782 732 B1**
(45) Date of publication and mention of the grant of the patent: **05.08.2009**
(21) Application number: 06023252.7
(22) Date of filing: 08.11.2006
(51) Int. Cl.: A61B 5/154

(54) **Blood-collecting needle set**
Nadelvorrichtung zum Blutsammeln
Dispositif d'aiguille de prélèvement de sang

(30) Priority: 08.11.2005 JP 2005323342
(43) Date of publication of application: 09.05.2007
(73) Proprietor: Nipro Corporation, Kita-ku, Osaka-shi, Osaka-fu, 531-8510 (JP)
(72) Inventor: Yashiro, Kenji, Tatebayashi-shi Gunma-ken 374-8518 (JP); Uchida, Yoshikuni, Tatebayashi-shi Gunma-ken 374-8518 (JP); Iida, Shinichirou, Tatebayashi-shi Gunma-ken 374-8518 (JP); Sekine, Shigeru, Osaka-shi Osaka-fu 531-8510 (JP)
(74) Representative: Banzer, Hans-Jörg

(56) References cited:
- EP-A1- 0 562 363
- EP-A2- 0 749 760
- US-A- 4 192 919
- US-A- 4 735 311
- US-A1- 2003 028 152

## Description

### [Technical Field]

This invention relates to a blood-collecting needle set.

### [Background Art]

To conduct the clinical examination such as serum examination and hemocyte examination, in general, a blood sample is collected by using a reduced-pressure blood-collecting instrument comprising a blood-collecting needle set and a reduced-pressure blood-collecting tube. The blood-collecting needle set comprises a blood-collecting needle assembly, a blood-collecting holder, a container, and a packaging bag. The blood-collecting needle assembly comprises a hub, and a needle body fixed to the hub in the state that the needle body inserted to the hub (see, for example, Japan Patent No.3144591). The hub is attached to the front end portion of the blood-collecting holder, and the blood-collecting tube is inserted therein from the rear-end opening of the blood-collecting holder. The container is attached to the hub and to the blood-collecting holder, and contains therein the needle body of a front side portion protruding forward from the hub. The blood-collecting needle assembly, the blood-collecting holder and the container are assembled and contained in the packaging bag, and are sterilized.

In the above blood-collecting needle set, the blood-collecting needle assembly, the blood-collecting holder and the container that are assembled together are contained in the packaging bag. Therefore, the blood-collecting needle set inclusive of the packaging bag as a whole possesses an increased volume arousing such a problem that a wide space is required for transportation and storage.

Further, by the time the blood-collecting needle set is prepared in the state of use, the operation must be conducted in three steps; i.e., open the packaging bag, take out the blood-collecting needle unit, the blood-collecting holder and the container that have been assembled from the packaging bag, and remove the container from the blood-collecting needle and the blood-collecting holder, which is troublesome.

Besides, the packaging bag is relatively large, and the volume of the medical waste becomes great correspondingly.

A blood-collection needle set according to the preamble of claim 1 is known from US 2003/0028152 A1.

### [Problems to be Solved by the Invention]

Problems to be solved by the invention are that a wide space is required for the transportation and storage, that a troublesome work is required by the time the blood-collecting needle set is prepared in the state of use, and that the volume of the medical waste becomes great. Therefore an object of the present invention is to provide a blood-collecting needle set that requires less space for transportation and storage; another object of the present invention is to provide a blood-collecting needle set that eliminates troublesome work preparing the needle set in the state of use; finally, another object of the present invention is to provide a blood-collecting needle set that eliminates a great volume of medical waste.

### [Means for Solving the Problems]

According to the present invention, these objects are achieved by a blood-collecting needle set as defined in claim 1. The dependent claims define preferred and advantageous embodiments of the invention.

It is an object of the present invention to provide a blood-collecting needle set capable of solving the above problems. To achieve the object, the feature of the invention resides in a blood-collecting needle set comprising a blood-collecting needle assembly comprising a hub, and a needle body fixed to the hub in the state that the needle body inserted to the hub; a blood-collecting holder comprising a front end portion which the hub is attached to , and a rear-end opening which a blood-collecting tube is inserted through; and a container detachably attached to the hub and to the blood-collecting holder, and contains therein the needle body of a front side portion protruding forward from the hub; wherein a seal closing the opening at the rear end of the blood-collecting holder is removably provided on the rear end surface of the blood-collecting holder.

The seal is often melt-adhered to the blood-collecting holder.

Further, a sealing label that can be broken is often clung to the outer surfaces of both the blood-collecting holder and of the container spanning across both outer surfaces thereof.

Furthermore, inner and outer cylindrical portions in an inner-and-outer-overlapped form are formed at the front end portion of the blood-collecting holder in a manner to protrude forward; the outer cylindrical portion protrudes forward beyond the inner cylindrical portion; the hub is inserted and screwed in the inner cylindrical portion; the rear part of the container is fitted in the outer cylindrical portion; and the inner peripheral surface of the outer cylindrical portion is intimately adhered to the outer peripheral surface at the rear portion of the container over the whole circumference.

Further, a ventilating groove is formed on the inner peripheral surface of the inner cylindrical portion in the axial direction.

Moreover, a circumferential protuberance is formed on either the inner peripheral surface of the outer cylindrical portion or the outer peripheral surface of the rear portion of the container, the circumferential protuberance protruding toward the other side and intimate adhering to the other side over the whole circumference.

Besides, plural circumferential protuberances are often arranged in the axial direction.

### [Effect of the Invention]

According to the present invention, because of using the seal without using the packaging bag, it is possible to decrease the volume of the blood-collecting needle set as a hole; to decrease the space for transportation and storage; to conduct the operation in only two steps, i.e., removing the seal, and taking out the container from the blood-collecting needle and the blood-collecting holder by the time the blood-collecting needle set is prepared in the state of use, therefore, saving troublesome work; and to be the volume of the medical waste small.

According to a preferred embodiment, because a trace of melt-adhesion remains on the blood-collecting holder when the seal is removed, it is possible for user to easily judge whether the blood-collecting needle set has not yet been used or is the used one.

According to another preferred embodiment, because the sealing label breaks when the container is taken out from the blood-collecting needle and the blood-collecting holder, it is possible for user to easily judge whether the blood-collecting needle set has not yet been used or is the used one.

Furthermore, it is possible to easily sterilize the interior of the blood-collecting needle set with a sterilizing gas.

Further, because the passage through which the external air enters into the blood-collecting needle set is not straight but is in a zig-zag shape due to the presence of the circumferential protuberance, it is difficult for the external air to enter into the blood-collecting needle set. Therefore, it is difficult for the bacteria present in the external air and bacteria adhered to the outer surface of the blood-collecting needle set to enter into the blood-collecting needle set. Therefore, it is difficult for the interior of the blood-collecting needle set to be contaminated with bacteria.

### [Brief Description of the Drawings]

Fig. 1 is a side view illustrating an embodiment of the present invention.
Fig. 2 is a plan view of Fig. 1.
Fig. 3 is a vertical side sectional view of Fig. 1.
Fig. 4 is a vertical side sectional view of Fig. 3 rotated by 45° in the axial direction.
Fig. 5 is a side view in a state where a container is removed from Fig. 1.
Fig. 6 is a vertical side sectional view of Fig. 4.
Fig. 7 is an enlarged view of a portion of Fig. 4.
Fig. 8 is a diagrammatic perspective view of a blood-collecting holder.

### [Best Mode for Carrying Out the Invention]

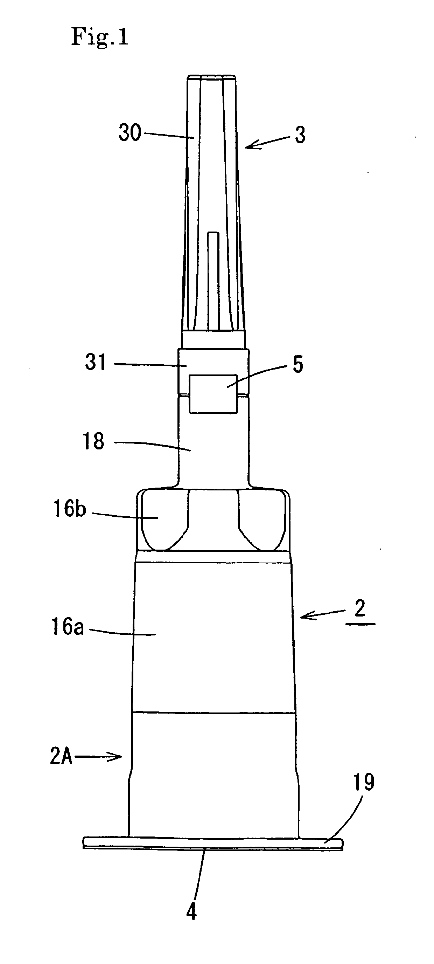

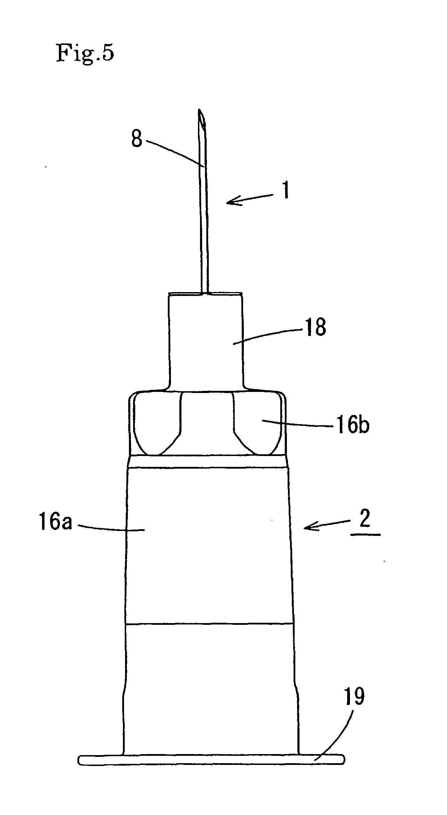

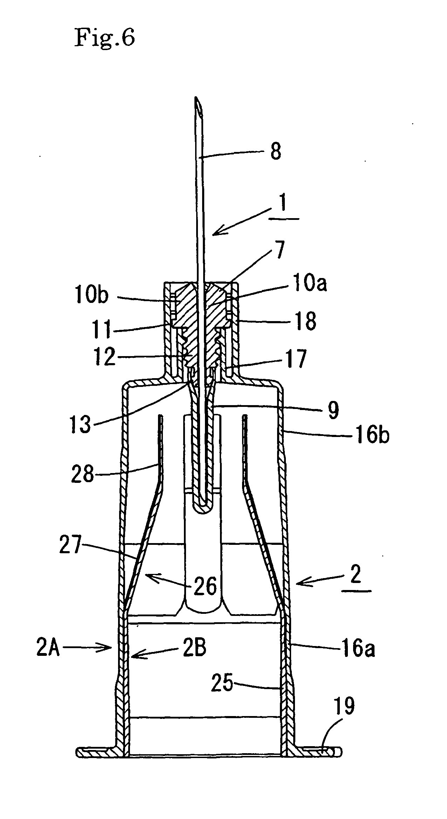

An embodiment in which the invention is applied to the blood-collecting needle set for multi-sampling will now be described with reference to the drawings. Referring to Figs. 1 to 6, the blood-collecting needle set comprises a blood-collecting needle assembly 1, a blood-collecting holder 2, a container 3, and a sterilizing seal (sterilizing paper) 4, and, in addition, comprises a sealing label 5.

In the invention, forward means the direction of a needlepoint side of the needle body 8 in the axial direction of the blood-collecting needle set, that is, an upper direction of Fig.1 and Figs.3 to 6. On the other hand, rearward means the direction of a flange portion 19 side to the needlepoint in the axial direction of the blood-collecting needle set, that is, the lower direction of Fig.1 and Figs.3 to 6.

Further, the front part (or portion, or side) means a forward part, and the rear part means a rearward part. The front end means a most forward part and, the rear end means a most rearward part.

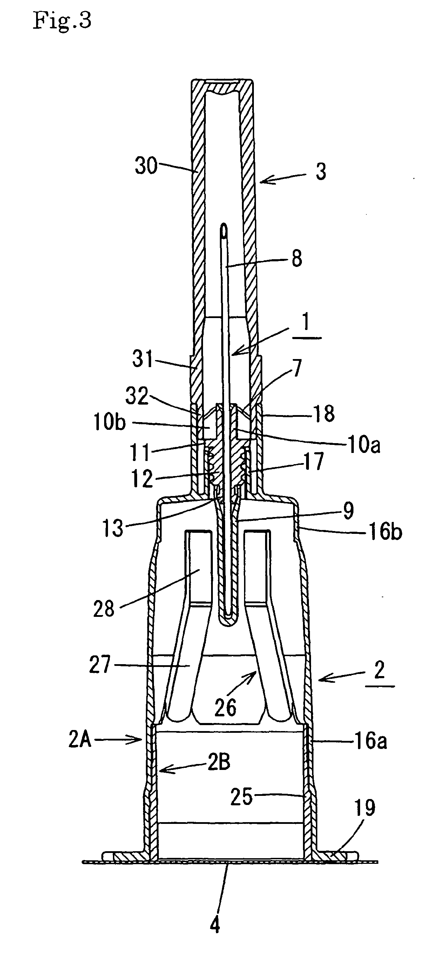

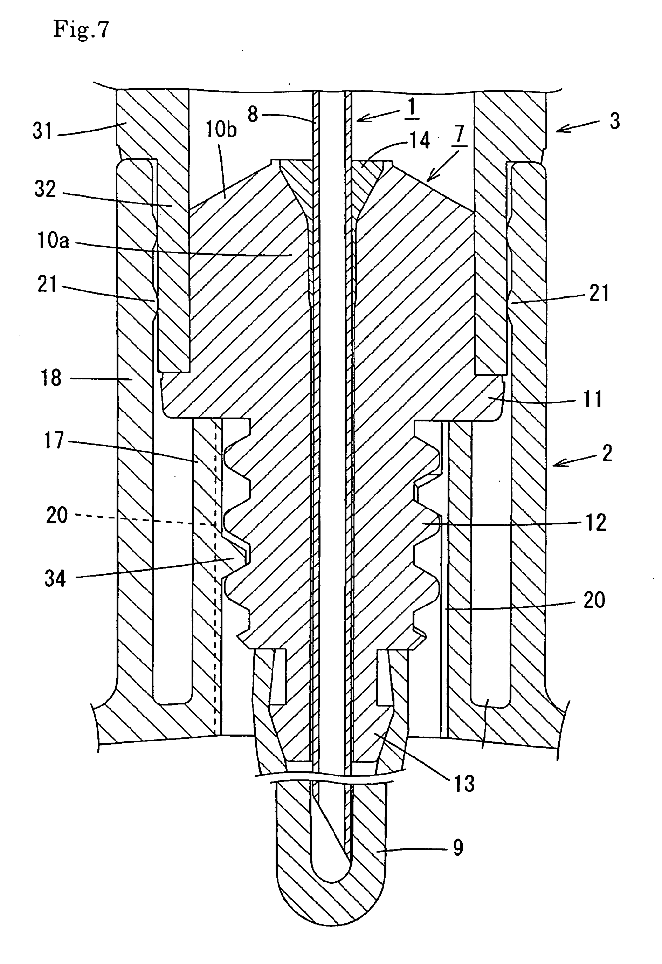

The blood-collecting needle assembly 1 comprises a hub (needle base) 7, a needle body 8 made of a stainless steel, and an elastic sheath (rubber sleeve) 9. The hub 7 is integrally molded by using a plastic material such as polypropylene. As shown in Figs.3 and 7, the hub 7 comprises a fitting portion 10, protruded portions 11, a male screw portion 12, and a mounting portion 13 in this order toward rearward. The fitting portion 10 is consisting of a hub main body 10a, and a wing-shaped part 10b formed to be protruded outward in the radial direction beyond the hub main body 10a as becoming symmetrical in the radial direction. The Protruded portions 11 protrude outward in the radial direction beyond the wing-shaped part 10b. The male screw portion 12 has a diameter smaller than the fitting portion 10 and forming a male screw on the outer peripheral portion thereof. The mounting portion 13 has a diameter smaller than the male screw portion 12. The needle body 8 is a hollow needle with the blade surfaces at the end of its both ends. The needle body 8 is inserted in the hub 7 and is fixed thereto with an adhesive 14. The elastic sheath 9 is made of an elastic material such as a rubber, is externally fitted to the mounting portion 13 of the hub 7, and covers the rear portion of the needle body 8.

A reduced-pressure blood-collecting tube is inserted in the blood-collecting holder 2 at the point of use. The blood-collecting holder 2 comprises a main body 2A and a holder portion 2B.

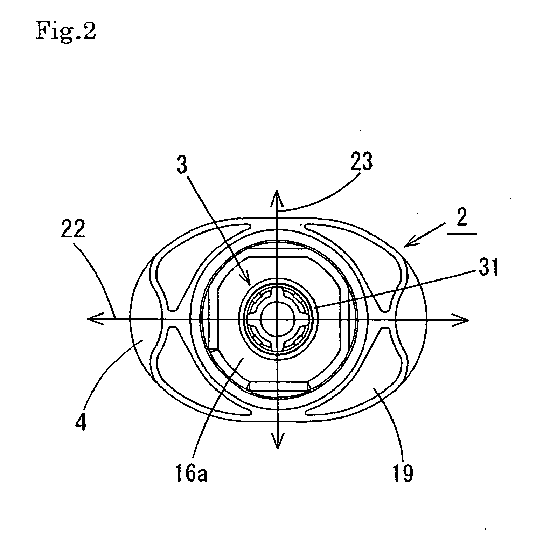

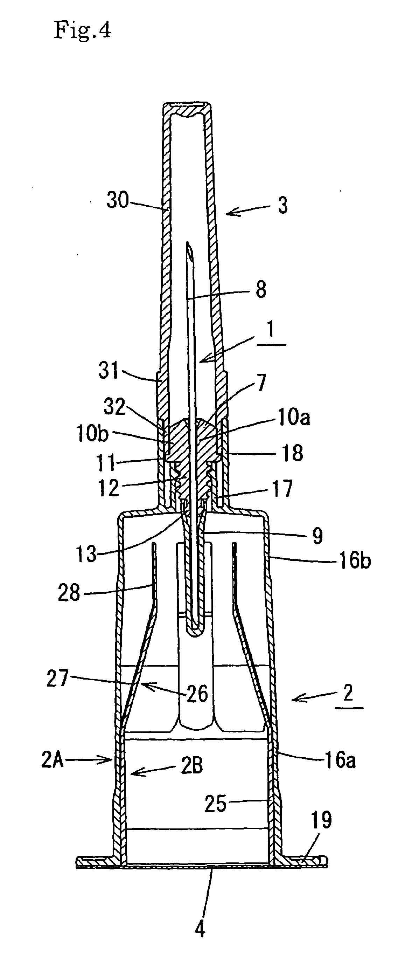

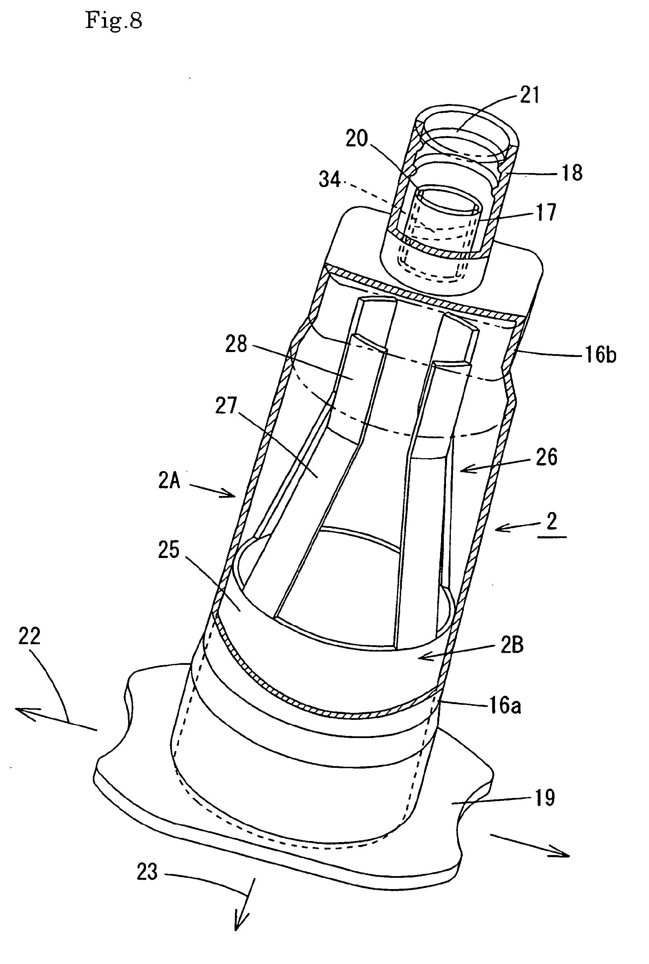

The main body 2A is integrally molded by using a plastic material such as polypropylene, is opening rear end. As also shown in Fig. 8, the main body 2A comprises a cylindrical portion 16a which is of a cylindrical shape having a constant diameter or a nearly constant diameter, a forward cylindrical portion 16b, an inner cylindrical portion 17 and an outer cylindrical portion 18, and a flange portion 19 protruding outward in the radial direction from the rear end of the cylindrical portion 16a. The forward cylindrical portion 16b is formed at the front end portion of the cylindrical portion 16a and is of a cylindrical shape. The shape of the section of the forward cylindrical portion 16a is a polygon, a polygonal rounded off a corner, or a round (shaped as square corner has rounded in this embodiment). The inner cylindrical portion 17 and outer cylindrical portion 18 are formed protruding forward beyond the front end of the forward cylindrical portion 16b. The inner cylindrical portion 17 and outer cylindrical portion 18 are of a cylindrical shape in concentric in an inner-and-outer-overlapped form. The front end of the forward cylindrical portion 16b gradually becomes partially closed so that the diameter of the opening at the front end of the forward cylindrical portion 16b is narrowed. The front end of the forward cylindrical portion 16b connects to the inner cylindrical portion 17 and the outer cylindrical portion 18. A female screw portion 34 is provided in the inner cylindrical portion 17. The male screw portion 12 of the hub 7 is inserted and screwed in the inner cylindrical portion 17, and the female screw portion 34 is connecting with the hub 7. Therefore, the hub 7 is detachably attached to the inner cylindrical portion 17. Further, as also shown in Figs. 3 and 4, when the male screw portion 12 of the hub 7 is inserted and screwed in the inner cylindrical portion12, the front part of the needle body 8 is protruding forward beyond the outer cylindrical portion 18, and at that time, the rear part of the needle body 8 and the elastic sheath 9 are protruding into the cylindrical portion 16a of the main body 2A. As also shown in Fig. 7, at least one or more ventilating grooves 20 for sterilizing gas are formed in the inner peripheral surface of the inner cylindrical portion 17 in the axial direction over the total length. The outer cylindrical portion 18 protrudes forward beyond the inner cylindrical portion 17. A circumferential protuberance 21 is formed to protrude inward of the diameter direction, and is formed so that the outer contour line in axial direction of the circumferential protuberance 21 is the arc. The circumferential protuberances 21 are arranged in a plural number on the inner peripheral surface of the protruded portion in the axial direction. The flange portion 19 is for easily holding the blood-collecting holder 2. In the flange portion 19 as shown in Fig. 2, a predetermined radial direction is a radial direction 22 of a maximum protrusion, and the symmetrical portions of the flange portion 19 in the radial direction 22 of the maximum protrusion are more greatly protruding in the radial direction than the other portions. In the flange portion 19, further, the radial direction at right angles with the radial direction 22 of the maximum protrusion is the direction 23 of a minimum protrusion and in which the symmetrical portions about the direction 23 of a minimum protrusion are a minimum in the flange portion 19 concerning the amount of protrusion outward in the radial direction from the cylindrical portion 16a. When the blood-collecting needle assembly 1 is attached to the blood-collecting holder 2, the blade surface at the front end of the blood-collecting needle assembly 1 is directed to either one of the minimum protrusions in the direction 23.

The holder portion 2B is attached in the main body 2A, and is integrally molded by using a plastic material such as polypropylene that has flexibility. The holder portion 2B is a cylindrical shape of a constant diameter or a nearly constant diameter. The holder portion 2B comprises a cylindrical portion 25 opening in the back-and-forth direction and a plurality of (four in this embodiment) flexible portions 26 arranged at the front end portion of the cylindrical portion 25 equally in the circumferential direction. The cylindrical portion 25 is fitted and is adhered or screwed into the rear portion of the cylindrical portion 16a of the main body 2A so as to be integral with the main body 2A. The flexible portion 26 protrudes forward beyond the front end of the cylindrical portion 25, surrounds the reduced-pressure blood-collecting tube, and is allowed to deform elastically in the radial direction. The flexible portion 26 is dogleg, and comprises an inclined portion 27 and a maintaining portion 28. The inclined portion 27 is the rear side of the flexible portion 26, and is inclined inward in the radial direction as it goes forward. The maintaining portion 28 protrudes forward from the front end of the inclined portion 27 and maintains the reduced-pressure blood-collecting tube. The maintaining portion 28 is in parallel or in nearly parallel with the axis of the holder portion 2B but is often inclined outward (or inward) in the radial direction as it goes forward.

The container 3 is integrally molded by using a plastic material such as polypropylene, is of a cylindrical shape having bottom which is opened at rearward, and may be the one that can be used as a protector of needlepoint. The container 3 is attached at its rear portion while placed between the wing-shaped part 10b of the hub 7 and the outer cylindrical portion 18. The container 3 is containing at least the needle body 8 of a front side portion protruding forward from the hub 7, and may be containing the fitting portion 10 of the hub 7 partly or fully. The container 3 comprises a holding portion 30 that forms majority of the portion of the container 3, a projecting portion 31 formed as having more larger diameter than the holding portion 30, and an attaching portion 32 formed as having more smaller diameter than the projecting portion 31 in this order toward rearward. The contour line on the inside of these portions in cross section is circular, and the inner diameter is equal or nearly equal to the outer diameter of the inner cylindrical portion 17. The holding portion 30 is that four salients attached equiangularly on the outer of a cylindrical part along with the total length of the holding portion 30,so that the holder portion 30 can be easily held by a person (operator) who collects blood. The projecting portion 31 is of a cylindrical shape having the same or nearly the same diameter as the outer cylindrical portion 18, and its rear end surface is in contact with the front end surface of the outer cylindrical portion 18 in the axial direction. The attaching portion 32 is of a cylindrical shape, and its outer diameter is equal or nearly equal to the inner diameter of the outer cylindrical portion 18. The attaching portion 32 is detachably attached between the fitting portion 10 of the hub 7 and the front part of the outer cylindrical portion 18, is detachably and externally fitted to the outward surface of the wing-shaped part 10b of the fitting portion 10 in the radial direction, and the rear end surface of the attaching portion 32 is in contact with the protruded portion 11 of the hub 7 in the axial direction. The attaching portion 32 is, further, removably inserted in the outer cylindrical portion 18, and the outer peripheral surface of the attaching portion 32 is intimately adhered to the inner peripheral surface of the outer cylindrical portion 18 over the whole circumference. Concretely, the circumferential protuberance 21 of the outer cylindrical portion 18 is intimately adhered to(contacted with) the outer peripheral surface of the attaching portion 32 being pushed thereon over the whole circumference. At the time of accomplishing the adhering, the circumferential protuberance 21 may or may not be elastically deformed. Further, the circumferential protuberance 21 may often be formed on the outer peripheral surface of the attaching portion 32 without being formed on the outer cylindrical portion 18.

The sterilizing seal 4 is removably fixed to the rear end surface of the flange portion 19 of the blood-collecting holder 2 and is sealing the rear end opening of the blood-collecting holder 2 in a manner that the rear-end opening of the flange portion 19 can be opened. The means for the above fixing, there can be employed melt-adhesion, adhesion using an adhesive or sticking by using a sticking agent. However, when melt-adhesion is employed, it is possible to easily judge whether the blood-collecting needle set has not yet been used or is the used one, because melt-adhesion leaves a trace of melt-adhesion on the rear end surface of the flange portion 19 on removing the sterilizing seal 4. The sterilizing seal 4 blocks the passage of bacteria but permits the permeation of a sterilizing gas of a high pressure. An ordinary seal may often be used without using the sterilizing seal 4.

The sealing label 5 is clung to the outer peripheral surfaces of both the projecting portion 31 of the container 3 and of the outer cylindrical portion 18 of the blood-collecting holder 2 spanning across both outer surfaces thereof. When the container 3 is removed from the blood-collecting needle assembly 1 and the blood-collecting holder 2, the sealing label 5 can be broken to make it possible to easily judge whether the blood-collecting needle set has not been used yet or is the used one. The means for the above clinging, there can be employed melt-adhesion, adhesion using an adhesive or sticking by using a sticking agent.

At the time of production of the blood-collecting needle set of the above constitution, the blood-collecting needle assembly 1, the blood-collecting holder 2 and the container 3 are assembled and, thereafter, the sterilizing seal 4 and the sealing label 5 are fixed.

Next, the blood-collecting needle set is introduced into a sterilizing room and is sterilized with the sterilizing gas of a high pressure. At this moment, the sterilizing gas permeates the sterilizing seal 4, the interior of the blood-collecting holder 2, through the ventilating grooves 20 in the inner cylindrical portion 17, a gap between the rear end surface of the protruded portion 11 of the hub 7 and the front end surface of the inner cylindrical portion 17, a gap between the fitting portion 10 of the hub 7 and the attaching portion 32, and arrives at the interior of the container 3 to thereby sterilize the interior of the blood-collecting needle set.

At the time of transportation and storage of the blood-collecting needle set, the blood-collecting needle set is exposed to the external air. However, the rear-end opening of the blood-collecting holder 2 is sealed with the sterilizing seal 4, and there is no probability that bacteria enters into the blood-collecting needle set through the opening at the rear end.

However, at the above situation, it is probable that there is a fear the bacteria may infiltrate through a gap between the container 3 and the outer cylindrical portion 18 of the blood-collecting holder 2. In this constitution, however, the circumferential protuberances 21 are formed to protrude inward of the diameter direction, are formed so that the outer in axially section of the circumferential protuberances 21 draws the arc, are arranged in a plural number on the inner peripheral surface of the protruded portion in the axial direction, and are, thus, intimately adhered onto the outer peripheral surface of the attaching portion 32 of the container 3 over the whole circumference. Due to the presence of the circumferential protuberances 21, therefore, the passage through which the external air enters into the blood-collecting needle set is not straight but is in a zig-zag form. As a result, it is more difficult for the external air to enter into the blood-collecting needle set. Accordingly, it is more difficult for the bacteria present in the external air and the bacteria adhered to the outer surfaces of the blood-collecting needle set to infiltrate into the blood-collecting needle set, and therefore it becomes difficult for the interior of the blood-collecting needle set to be contaminated with bacteria.

At the time of the use of the blood-collecting needle set, the sterilizing seal 4 is removed and, thereafter, the container 3 is taken away from the blood-collecting needle 1 and the blood-collecting holder 2. At this moment, the container 3 is not turned but is pulled in a manner to be separated away forward from the blood-collecting needle 1 and the blood-collecting holder 2, and it becomes easy to break the sealing label 5. Thus, the container 3 can be taken out from the blood-collecting needle 1 and the blood-collecting holder 2, and the blood-collecting needle set is ready to be the state of use.

Next, the person (operator) who collects the blood stabs the blood-collecting needle 1 into a vein in the arm or other part of a person from whom the blood is to be collected. At this moment, it is necessary to make sure the direction of the blade surface at the front end of the needle body 8 of the blood-collecting needle assembly 1 and to use it with the blade surface being faced upward. Here, however, when the blood-collecting needle is screw-fitted to the conventional blood-collecting holder, a relative positional relationship is not steadily constant between the blade surface and the blood-collecting holder in the circumferential direction. Therefore, the blade surface is not directed to either side of the flange portion in the radial direction of the minimum protrusion which is provided for easy grip of the blood-collecting holder, but is often directed in an intermediate radial direction of the flange portion between the direction of the maximum protrusion and the direction of the minimum protrusion. In such a case, the flange portion 19 gets in touch with the arm or other part of the person from whom the blood is being collected and, therefore, the stab angle of the needle body 8 is not decreased with respect to the vein arousing a problem of high probability of risk for damaging the blood vessel at the time of stabbing. With the above constitution, however, the blade surface is directed to either side in the direction 23 of the minimum protrusion, and the flange portion 19 does not disturb the operation for collecting the blood. Accordingly, the stab angle of the needle body 8 with respect to the vein can be decreased to lower the probability of risk of damaging the blood vessel at the time of stabbing.

In a state where the needle body 8 of the blood-collecting needle assembly 1 is stabbed into the vein, the rear-end opening of the needle body 8 is covered with the elastic sheath 9. Therefore, the blood does not leak into the blood-collecting holder 2 and does not contaminate the blood-collecting holder 2.

Next, the reduced-pressure blood-collecting tube (not shown) is inserted with its plug facing forward into the blood-collecting holder 2 through the opening at the rear end thereof. Therefore, as the reduced-pressure blood-collecting tube is inserted, the inclined portions 27 of the flexible portions 26 surrounding the plug deform elastically outward in the radial direction while guiding the plug, whereby the plug is inserted in a state of being positioned at the central portion of the blood-collecting holder 2 in the radial direction due to the flexible portions 26. Further, the maintaining portion 28 is inclined outward in the radial direction as it goes forward, and holds the plug. At this time, the maintaining portion 28 which is pushed and spread, is accommodated in the space which is rounded off the corner of the polygon inner of the forward cylindrical portion 16b.

As the reduced-pressure blood-collecting tube is inserted as described above, the plug compresses the elastic sheath 9, the rear part of the needle body 8 penetrates through the elastic sheath 9 and the plug, and a blood sample of a predetermined amount flows into the reduced-pressure blood-collecting tube depending upon the degree of pressure reduction of the pressure-reduced blood-collecting tube. Here, the pressure-reduced blood-collecting tube maintained by the maintaining portion 28 receives the force directed forward due to the returning force of the flexible portion 26. The force directed forward is cancelled by a force that pushes the reduced-pressure blood-collecting tube rearward produced by the compressed elastic sheath 9. Therefore, the reduced-pressure blood-collecting tube does not move rearward. Namely, there does not occur a kick-back phenomenon in that the plug escapes from the needle body 8.

When the blood sample of a predetermined amount is collected in the reduced-pressure blood-collecting tube as described above, the pressure in the reduced-pressure blood-collecting tube rises, and the collection of blood ends. After the end of blood collection, the reduced-pressure blood-collecting tube is forcibly pulled away from the blood-collecting holder 2. Therefore, the rear part of the needle body 8 is pulled away from the plug and from the elastic sheath 9. Then, the elastic sheath 9 returns back to the figure which is before the reduced-pressure blood-collecting tube is inserted, and the opening at the rear end of the needle body 8 is covered with the elastic sheath 9. Therefore, the blood does not leak into the blood-collecting holder 2, and does not contaminate the blood-collecting holder 2 or the reduced-pressure blood-collecting tube.

After the operation for collecting the blood, it is desired that the blood-collecting needle assembly 1 and the blood-collecting holder 2 are disposed of in a state of being attached together from the standpoint of preventing infection. In most of the hospitals, however, the blood-collecting needle assembly 1 which includes, as a constituent part, the needle body 8 which is a metal part and the blood-collecting holder 2 constituted by only the plastic material have, in practice, been disposed of separately because of the relationship of the cost for treating the wastes.

In the above example of constitution, however, the blood-collecting needle assembly 1 and the blood-collecting holder 2 are separably attached together by screw-fitting to cope with either the disposal in a state where the blood-collecting needle assembly 1 and the blood-collecting holder 2 are attached together or the separate disposal in a state where the blood-collecting needle 1 and the blood-collecting holder 2 are separated away from each other.

## Claims

1. A blood-collecting needle set comprising:
a blood-collecting needle assembly (1) comprising a hub (7), and a needle body (8) fixed to the hub (7) in the state in which the needle body (8) is inserted to the hub (7);
a blood-collecting holder (2) comprising a front end portion which the hub (7) is attached to , and a rear-end opening through which a blood-collecting tube is inserted; and
a container (3) detachably attached to the hub (7) and to the blood-collecting holder (2), said container (3) containing a front side portion of the needle body (8), said front side portion protruding forward from the hub (7);
a seal (4) closing the opening at the rear end of the blood-collecting holder (2) being removably provided on the rear end surface of the blood-collecting holder (2);
wherein inner and outer cylindrical portions (17, 18) in an inner-and-outer-overlapped form are formed at the front end portion of the blood-collecting holder (2) in a manner to protrude forward;
the outer cylindrical portion (18) protruding forward beyond the inner cylindrical portion (17);
the hub (7) being inserted and screwed in the inner cylindrical portion (17);
the rear part of the container (3) being fitted in the outer cylindrical portion (18) ;
the inner peripheral surface of the outer cylindrical portion (18) being intimately adhered to the outer peripheral surface at the rear portion of the container (3) over the whole circumference;
**characterized in that**
at least one ventilating groove (20) is formed straight on the inner peripheral surface of the inner cylindrical portion (17) in the axial direction; and
at least one circumferential protuberance (21) is formed on either the inner peripheral surface of the outer cylindrical portion (18) or the outer peripheral surface of the rear portion of the container (3), the circumferential protuberance (21) protruding toward the outer peripheral surface of the rear portion of the container (3) or the inner peripheral surface of the outer cylindrical portion (18), respectively, for intimately adhering to said respective surface over the whole circumference.

2. The blood-collecting needle set according to claim 1, wherein the seal (4) is melt-adhered to the blood-collecting holder (2).

3. The blood-collecting needle set according to claim 1 or 2, wherein a sealing label (5) that can be broken is clung to the outer surfaces of the blood-collecting holder (2) and of the container (3) spanning across both outer surfaces thereof.

4. The blood-collecting needle set according to any one of claims 1-3, wherein plural circumferential protuberances (21) are arranged in the axial direction.

5. The blood-collecting needle set according to any one of claims 1-4, wherein plural ventilating grooves (20) are formed straight on the inner peripheral surface of the inner cylindrical portion (17) in the axial direction.

## Patentansprüche

1. Nadelsatz zur Blutabnahme, umfassend:
eine Nadelanordnung (1) zur Blutabnahme, welche einen Mittelteil (7) und einen Nadelkörper (8) umfasst, welcher an dem Mittelteil (7) in dem Zustand, in welchem der Nadelkörper (8) in den Mittelteil (7) eingeführt ist, befestigt ist;
eine Haltevorrichtung (2) zur Blutabnahme, welche einen vorderen Endabschnitt, der an dem Mittelteil (7) angebracht ist, und eine Öffnung am hinteren Ende, durch welche ein Rohr zur Blutabnahme eingeführt ist, umfasst; und
einen Behälter (3), welcher lösbar an dem Mittelteil (7) und an der Haltevorrichtung (2) zur Blutabnahme angebracht ist, wobei der Behälter (3) einen vorderen Seitenabschnitt des Nadelkörpers (8) enthält, wobei der vordere Seitenabschnitt von dem Mittelteil (7) nach vorn gerichtet hervorragt;
wobei eine Dichtung (4), welche die Öffnung an dem hinteren Ende der Haltevorrichtung (2) zur Blutabnahme verschließt, entfernbar an der hinteren Endoberfläche der Haltevorrichtung (2) zur Blutabnahme vorhanden ist;
wobei ein innerer und ein äußerer Zylinderabschnitt (17, 18) in einer innen und außen überlappenden Form an dem vorderen Endabschnitt der Haltevorrichtung (2) zur Blutabnahme derart ausgebildet sind, dass sie nach vorn hervorragen;
wobei der äußere Zylinderabschnitt (18) nach vorn über den inneren Zylinderabschnitt (17) hervorragt;
wobei das Mittelteil (7) in dem inneren Zylinderabschnitt (17) eingeführt und verschraubt ist;
wobei der hintere Teil des Behälters (3) in den äußeren Zylinderabschnitt (18) eingepasst ist;
wobei die innere Umfangsfläche des äußeren Zylinderabschnitts (18) unmittelbar an der äußeren Umfangsfläche an dem hinteren Abschnitt des Behälters (3) über den gesamten Umfang hinweg haftet;
**dadurch gekennzeichnet,**
**dass** zumindest eine Lüftungsvertiefung (20) gerade auf der inneren Umfangsfläche des inneren Zylinderabschnitts (17) in der axialen Richtung ausgebildet ist; und
**dass** zumindest eine Umfangsausstülpung (21) entweder auf der inneren Umfangsfläche des äußeren Zylinderabschnitts (18) oder auf der äußeren Umfangsfläche des hinteren Abschnitts des Behälters (3) ausgebildet ist, wobei die Umfangsausstülpung (21) zu der äußeren Umfangsfläche des hinteren Abschnitts des Behälters (3) bzw. zu der inneren Umfangsfläche des äußeren Zylinderabschnitts (18) hervorragt, um unmittelbar über dem gesamten Umfang an der entsprechenden Fläche zu haften.

2. Nadelsatz zur Blutabnahme nach Anspruch 1, **dadurch gekennzeichnet, dass** die Dichtung (4) mittels Warmkleber an der Haltevorrichtung (2) zur Blutabnahme angebracht ist.

3. Nadelsatz zur Blutabnahme nach Anspruch 1 oder 2, **dadurch gekennzeichnet, dass** ein Dichtungskennzeichen (5), welches aufgebrochen werden kann, an der äußeren Oberfläche der Haltevorrichtung (2) zur Blutabnahme und an der äußeren Oberfläche des Behälters (3) anhaftet, wobei es beide äußeren Oberflächen überspannt.

4. Nadelsatz zur Blutabnahme nach einem der Ansprüche 1-3, **dadurch gekennzeichnet, dass** mehrere Umfangsausstülpungen (21) in der axialen Richtung angeordnet sind.

5. Nadelsatz zur Blutabnahme nach einem der Ansprüche 1-4, **dadurch gekennzeichnet, dass** mehrere Lüftungsvertiefungen (20) gerade auf der inneren Umfangsfläche des inneren Zylinderabschnitts (17) in der axialen Richtung ausgebildet sind.

## Revendications

1. Dispositif d'aiguille pour prélèvement de sang comprenant :
un ensemble d'aiguille pour prélèvement de sang (1) comprenant une garde (7) et un corps d'aiguille (8) fixé à la garde (7) dans l'état où le corps d'aiguille (8) est inséré dans la garde (7) ;
un support de prélèvement de sang (2) comprenant une partie d'extrémité avant, à laquelle est fixée la garde (7) et une ouverture dans l'extrémité arrière à travers laquelle est inséré un tube de prélèvement de sang ; et
un récipient (3) fixé de manière détachable à la garde (7) et au support de prélèvement de sang (2), ledit récipient (3) contenant une partie latérale avant du corps d'aiguille (8), ladite partie latérale avant saillant vers l'avant depuis la garde (7) ;
un joint (4) fermant l'ouverture à l'extrémité arrière du support de prélèvement de sang (2), installé de manière amovible sur la surface de l'extrémité arrière du support de prélèvement de sang (2) ; dans lequel les parties cylindriques intérieure et extérieure (17, 18) ayant une forme à recouvrement intérieur et extérieur, sont ménagées dans la partie d'extrémité avant du support de prélèvement de sang (2), de façon à faire saillie vers l'avant ;
la partie cylindrique extérieure (18) saillant vers l'avant au delà de la partie cylindrique intérieure (17) ;
la garde (7) étant insérée et vissée dans la partie cylindrique intérieure (17) ;
la partie arrière du récipient (3) étant fixée dans la partie cylindrique extérieure (18) ;
la surface périphérique intérieure de la partie cylindrique extérieure (18) étant intimement fixée à la surface périphérique extérieure au niveau de la partie arrière du récipient (3) sur toute la circonférence ;
**caractérisé en ce que**
au moins une cannelure de ventilation (20) est ménagée directement sur la surface périphérique interne de la partie cylindrique intérieure (17) dans le sens axial ; et
au moins une saillie circonférentielle (21) est ménagée sur la surface périphérique interne de la partie cylindrique extérieure (18) ou la surface périphérique extérieure de la partie arrière du récipient (3), la saillie circonférentielle (21) saillant vers la surface périphérique extérieure de la partie arrière du récipient (3) ou la surface périphérique intérieure de la partie cylindrique extérieure (18), respectivement, pour adhérer intimement à ladite surface respective sur toute la circonférence.

2. Dispositif d'aiguille pour prélèvement de sang selon la revendication 1, dans lequel le joint (4) est fixé par fusion au support de prélèvement de sang (2).

3. Dispositif d'aiguille pour prélèvement de sang selon les revendications 1 ou 2, dans lequel une étiquette de scellement (5) qui peut être rompue est fixée sur les surfaces extérieures du support de prélèvement de sang (2) et du récipient (3) qui s'étend à travers ses deux surfaces.

4. Dispositif d'aiguille pour prélèvement de sang selon l'une quelconque des revendications 1-3, dans lequel plusieurs saillies circonférentielles (21) sont disposées dans le sens axial.

5. Dispositif d'aiguille pour prélèvement de sang selon l'une quelconque des revendications 1-4, dans lequel des cannelures de ventilation (20) sont ménagées directement sur la surface périphérique de la partie cylindrique intérieure (17), dans le sens axial.
